# EUROPEAN PATENT APPLICATION

(11) **EP 4 379 741 A1**
(43) Date of publication of application: **05.06.2024**
(21) Application number: 23837931.7
(22) Date of filing: 14.02.2023
(51) Int. Cl.: G16H 50/50, G16H 50/70, G16H 10/60, G16H 40/60, G16H 30/20, A61B 5/00, A61B 5/024, A61B 5/145, A61B 3/14

(54) **DEVICE AND METHOD FOR PREDICTING MYOPIA REGRESSION**

(30) Priority: 12.10.2022 KR 20220130653
(71) Applicant: Aivis Inc., Seoul 06236 (KR); Lee, Seong Jun, Daejeon 35233 (KR)
(72) Inventor: LEE, Seong Jun, Daejeon 35233 (KR); LEE, Dae Hong, Seoul 07318 (KR); YE, Jae Hyung, Seoul 06218 (KR); LEE, Kwang Yeol, Seongnam-si, Gyeonggi-do 13562 (KR)
(74) Representative: Isarpatent
(86) International application number: PCT/KR2023/002165
(87) International publication number: WO 2024/080459

(57) **Abstract**

Provided is a medical apparatus for predicting myopic regression. The medical apparatus for predicting myopic regression can be configured to predict at least one of a probability of myopic regression and whether myopic regression will occur from numerical data including a refractive power, a corneal curvature, an eye axial length, a photopic pupil size, a mesopic pupil size, a corneal diameter, a corneal thickness, a corneal epithelial thickness, a high-order aberration, a visual acuity, an intraocular pressure, a sex, and an age using a machine learning processor.

## Description

### [Technical Field]

The present disclosure relates to an apparatus and method for predicting myopic regression, and more specifically, to an apparatus and method for predicting myopic regression using machine learning.

### [Background Art]

Myopic regression is a situation in which a patient's eyesight deteriorates again after vision correction surgery such as laser-assisted in situ keratomileusis (LASIK), laser-assisted sub-epithelial keratectomy (LASEK), small incision lenticule extraction (SMILE), etc. Myopic regression frequently occurs within one week to six months after surgery, and may occur several years after surgery depending on eye care after surgery. Currently, it is not possible to objectively provide the probability of myopic regression to a patient, and thus there is a reliability issue between the patient and doctor. Corneal collagen cross-linking is a technology in which riboflavin is used as a photosensitizer and then ultraviolet light A is used to cause a photochemical reaction. The photochemical reaction promotes formation of intra- and interfibrillar covalent bonds or cross-links in the corneal stroma, thereby strengthening the corneal tissue. When there is a high probability of myopic regression, it is possible to minimize the myopic regression using corneal collagen cross-linking. However, currently, because a prediction of myopic regression cannot be calculated as an objective number and the degree of risk is determined on the basis of subjective judgment of doctors, the consistency of predicting myopic regression may degrade depending on differences between doctors. Accordingly, patients may feel anxious about vision correction surgery, and their confidence in surgical methods presented to them may decrease. Therefore, an apparatus and method for objectively predicting the probability of myopic regression are necessary.

### [Disclosure]

### [Technical Problem]

The present disclosure is directed to providing an apparatus and method for objectively predicting at least one of a probability of myopic regression and whether myopic regression will occur.

### [Technical Solution]

One aspect of the present disclosure provides an apparatus for predicting myopic regression which is configured to predict at least one of a probability of myopic regression and whether myopic regression will occur from numerical data including a refractive power (a degree of myopia or astigmatism), a corneal curvature, an eye axial length, a photopic pupil size, a mesopic pupil size, a corneal diameter, a corneal thickness, a corneal epithelial thickness, a high-order aberration, a visual acuity, an intraocular pressure, a sex, and an age, using a machine learning processor.

To predict at least one of the probability of myopic regression and whether myopic regression will occur, a deep learning processor and image data including a captured fundus image, a corneal endothelial cell image, a corneal shape and aberration analyzer (e.g., Keratron Scout) image, an optical coherence tomography image, an optical path difference (OPD)-scan III image, and a computed corneal tomography machine (e.g., Pentacam AXL) image may be further used.

The machine learning processor may be configured to extract a first feature from the numerical data, the deep learning processor may be configured to extract a second feature from the image data, and the apparatus for predicting myopic regression may include a fusion processor configured to predict at least one of the probability of myopic regression and whether myopic regression will occur from the first feature and the second feature.

The deep learning processor may include a first sub-model processor configured to extract a first sub-feature from the captured fundus image, a second sub-model processor configured to extract a second sub-feature from the corneal endothelial cell image, a third sub-model processor configured to extract a third sub-feature from the corneal shape and aberration analyzer image, a fourth sub-model processor configured to extract a fourth sub-feature from the optical coherence tomography image, a fifth sub-model processor configured to extract a fifth sub-feature from the OPD-scan III image, a sixth sub-model processor configured to extract a sixth sub-feature from the computed corneal tomography machine image, and a sub-fusion processor configured to extract the second feature from the first to sixth sub-features.

The machine learning processor may include a missing value processing processor configured to process a missing value of the numerical data, a preprocessing processor configured to preprocess the numerical data, a learning processor configured to extract a feature from the preprocessed numerical data, and a postprocessing processor configured to predict at least one of the probability of myopic regression and whether myopic regression will occur from the extraction unit.

Another aspect of the present disclosure provides a method of predicting myopic regression including predicting at least one of a probability of myopic regression and whether myopic regression will occur from numerical data including a refractive power, a corneal curvature, an eye axial length, a photopic pupil size, a mesopic pupil size, a corneal diameter, a corneal thickness, a corneal epithelial thickness, a high-order aberration, a visual acuity, an intraocular pressure, a sex, and an age, using a machine learning processor.

The predicting of at least one of the probability of myopic regression and whether myopic regression will occur may further use a deep learning processor and image data including a captured fundus image, a corneal endothelial cell image, a corneal shape and aberration analyzer image, an optical coherence tomography image, an OPD-scan III image, and a computed corneal tomography machine image.

The predicting of at least one of the probability of myopic regression and whether myopic regression will occur may include extracting a first feature from the numerical data using the machine learning processor, extracting a second feature from the image data using the deep learning processor, and predicting at least one of the probability of myopic regression and whether myopic regression will occur from the first feature and the second feature.

The extracting of the second feature may include extracting a first sub-feature from the captured fundus image, extracting a second sub-feature from the corneal endothelial cell image, extracting a third sub-feature from the corneal shape and aberration analyzer image, extracting a fourth sub-feature from the optical coherence tomography image, extracting a fifth sub-feature from the OPD-scan III image, extracting a sixth sub-feature from the computed corneal tomography machine image, and extracting the second feature from the first to sixth sub-features.

Another aspect of the present disclosure provides a method of training a myopic regression prediction apparatus configured to predict at least one of a probability of myopic regression and whether myopic regression will occur from numerical data including a refractive power, a corneal curvature, an eye axial length, a photopic pupil size, a mesopic pupil size, a corneal diameter, a corneal thickness, a corneal epithelial thickness, a high-order aberration, a visual acuity, an intraocular pressure, a sex, and an age, the method including generating, by at least one of a missing value processing method, a preprocessing method, and a postprocessing method, a plurality of different machine learning processors, training the plurality of machine learning processors, evaluating the plurality of machine learning processors, and selecting an optimal machine learning processor among the plurality of machine learning processors on the basis of evaluation results.

### [Advantageous Effects]

According to the technical spirit of the present disclosure, it is possible to objectively predict at least one of the probability of myopic regression and whether myopic regression will occur. Accordingly, patients can be provided with objective prediction results, and trust between patients and doctors can be increased. Also, when the probability of myopic regression is high, a surgery method, such as corneal collagen cross-linking, may be used.

Effects obtainable from exemplary embodiments of the present disclosure are not limited to those described above, and other effects which have not been described may be clearly derived and understood by those skilled in the art to which the exemplary embodiments of the present disclosure pertain from the following description. In other words, unintended effects according to the implementation of the exemplary embodiments of the present disclosure may also be derived by those skilled in the art from the exemplary embodiments of the present disclosure.

### [Description of Drawings]

FIG. 1 is a block diagram of a myopic regression prediction apparatus according to embodiments of the present disclosure.
FIG. 2 is a block diagram of a myopic regression prediction apparatus according to embodiments of the present disclosure.
FIG. 3 is a block diagram of a deep learning processor according to embodiments of the present disclosure.
FIG. 4 is a flowchart of a myopic regression prediction method according to embodiments of the present disclosure.
FIG. 5 is a flowchart of a myopic regression prediction method according to embodiments of the present disclosure.
FIG. 6 is a flowchart of an operation of extracting a second feature from image data using a deep learning processor according to embodiments of the present disclosure.
FIG. 7 is a flowchart of a method of training a myopic regression prediction apparatus according to embodiments of the present disclosure.

### [Modes of the Invention]

Hereinafter, embodiments of the present disclosure will be described in detail with reference to the accompanying drawings.

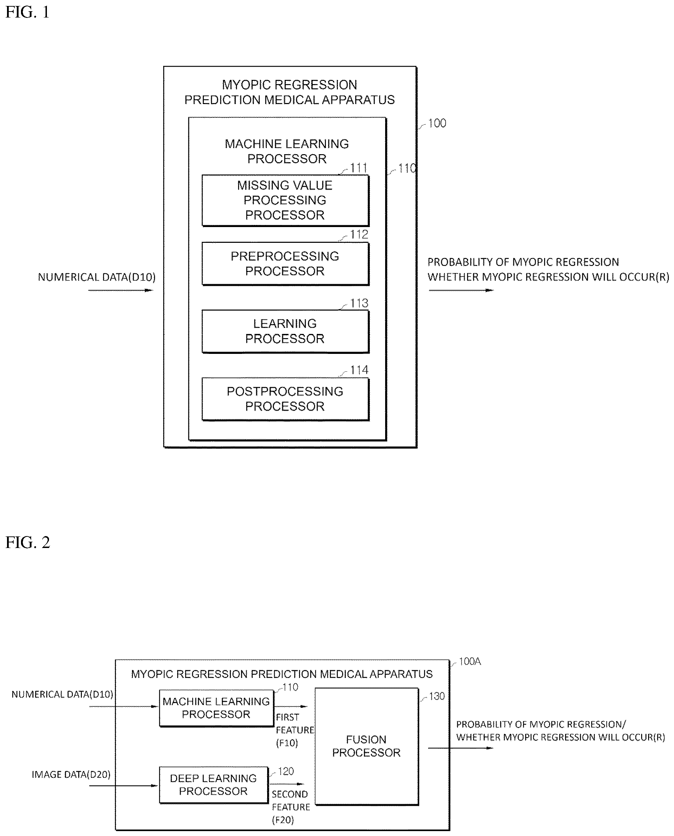

FIG. 1 is a block diagram of a myopic regression prediction apparatus 100 according to embodiments of the present disclosure.

Referring to FIG. 1, the myopic regression prediction apparatus 100 may include a machine learning processor 110. The machine learning processor 110 may predict at least one R of the probability of myopic regression and whether myopic regression will occur from numerical data D10.

The numerical data D10 may include a refractive power, a corneal curvature, an eye axial length, a photopic pupil size, a mesopic pupil size, a corneal diameter, a corneal thickness, a corneal epithelial thickness, a high-order aberration, a visual acuity, an intraocular pressure, a sex, and an age. The sex is categorical data but may be converted into numerical data by defining females as 0 and males as 1. In reverse, females may be defined as 1, and males may be defined as 0. Description of each piece of the numerical data D10 is self-evident to those skilled in the field of ophthalmology and thus will be omitted. When the numerical data D10 is used, the at least one R of the probability of myopic regression and whether myopic regression will occur can be predicted with high accuracy. The numerical data D10 is not limited to that listed herein, and numerical data which is not listed herein may be added to improve the accuracy of predicting the at least one R of the probability of myopic regression and whether myopic regression will occur.

The machine learning processor 110 may include a missing value processing processor 111, a preprocessing processor 112, a learning processor 113, and a postprocessing processor 114. The missing value processing processor 111, the preprocessing processor 112, the learning processor 113, and the postprocessing processor 114 may be processors each configured as different pieces of hardware. However, the missing value processing processor 111, the preprocessing processor 112, the learning processor 113, and the postprocessing processor 114 of the present disclosure are not limited thereto and may be configured as one piece of hardware and perform different functions.

The learning processor 113 may be configured to perform arbitrary machine learning. For example, the learning processor 113 may perform machine learning on the basis of a linear regression model, a logistic regression model, a support vector machine (SVM) model, a decision tree model, a random forest model, an extreme gradient boosting (XGBoost) model, or a deep learning model. In the present specification, machine learning includes deep learning. According to some embodiments, the learning processor 113 may use an ensemble, that is, a combination, of a plurality of machine learning methods.

According to some embodiments, the machine learning processor 110 may include the missing value processing processor 111. The missing value processing processor 111 may process a missing value of the numerical data D10. The missing value processing processor 111 may perform an arbitrary missing value processing method. For example, the missing value processing method may be do nothing, k nearest neighbor (K-NN), multivariate imputation by chained equation (MICE), missForest, AutoEncoder, generative adversarial imputation nets (GAIN), expectation maximization (EM), or a combination thereof.

According to some embodiments, the machine learning processor 110 may include the preprocessing processor 112. The preprocessing processor 112 may preprocess the numerical data D10 of which the missing value has been processed. The preprocessing processor 112 may perform an arbitrary preprocessing method. For example, the preprocessing method may be standardization, normalization, principal component analysis (PCA), fast independent component analysis (ICA), or a combination thereof.

The learning processor 113 may extract a feature from the preprocessed numerical data D10.

According to some embodiments, the machine learning processor 110 may include the postprocessing processor 114. The postprocessing processor 114 may postprocess the feature extracted by the learning processor 113 and output the at least one R of the probability of myopic regression and whether myopic regression will occur. The postprocessing processor 114 may perform an arbitrary postprocessing method. For example, the postprocessing method may be no postprocessing, sigmoid, isotonic, or a combination thereof.

The myopic regression prediction apparatus 100 may output the at least one R of the probability of myopic regression and whether myopic regression will occur. According to some embodiments, whether myopic regression will occur may be calculated from the probability of myopic regression. For example, when the probability of myopic regression exceeds 0.5, it may be predicted that myopic regression will occur, and when the probability of myopic regression is 0.5 or less, it may be predicted that myopic regression will not occur. The threshold value of 0.5 may be arbitrarily changed.

FIG. 2 is a block diagram of a myopic regression prediction medical apparatus 100A according to embodiments of the present disclosure.

Referring to FIG. 2, the myopic regression prediction medical apparatus 100A may further use image data D20 and a deep learning processor 120.

Here, the image data D20 may include a captured fundus image, a corneal endothelial cell image, a corneal shape and aberration analyzer (e.g., Keratron Scout) image, an optical coherence tomography image, an optical path difference (OPD)-scan III image, and a computed corneal tomography machine (e.g., Pentacam AXL) image. Description of each piece of the image data D20 is self-evident to those skilled in the field of ophthalmology and thus will be omitted. When the image data D20 is used, at least one R of the probability of myopic regression and whether myopic regression will occur can be predicted with high accuracy. The image data D20 is not limited to that listed herein, and image data which is not listed herein may be added to improve the accuracy of predicting the at least one R of the probability of myopic regression and whether myopic regression will occur. The image data D20 may be in the form of a two-dimensional (2D) or three-dimensional (3D) array of numbers.

The deep learning processor 120 may perform learning and data inference on the basis of a deep learning model including a convolution-based deep neural network. The convolution-based deep neural network is a deep neural network including a layer that performs a convolution operation. Detailed description of a convolution operation is self-evident to those skilled in the field of machine learning and thus will be omitted. For example, the convolution-based deep neural network may be a LeNet, an AlexNet, a VGGNet, a GoogleNet, or a ResNet. However, the convolution-based deep neural network is not limited to those listed herein and may include an arbitrary deep neural network including a convolution layer.

The myopic regression prediction medical apparatus 100A may include a machine learning processor 110, the deep learning processor 120, and a fusion processor 130. The machine learning processor 110 may extract a first feature F10 from numerical data D10, and the deep learning processor 120 may extract a second feature F20 from the image data D20. Here, a feature indicates a number or an array of numbers.

The fusion processor 130 may predict the at least one R of the probability of myopic regression and whether myopic regression will occur. The fusion processor 130 may include, for example, a fully connected layer.

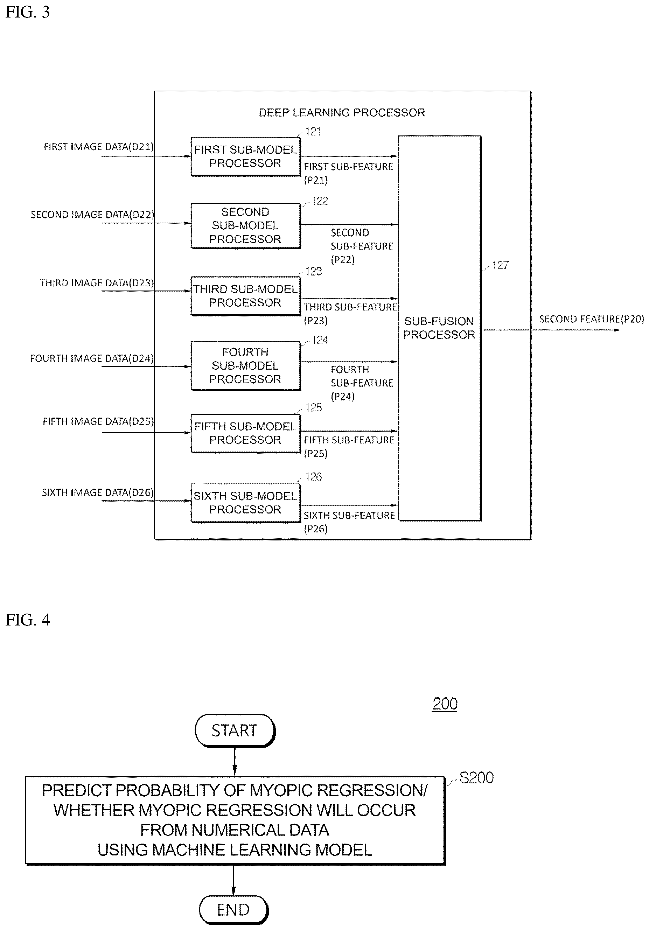

FIG. 3 is a block diagram of the deep learning processor 120 according to embodiments of the present disclosure.

Referring to FIG. 3, the deep learning processor 120 may include first to sixth sub-model processors 121 to 126 and a sub-fusion processor 127. FIG. 3 shows that the deep learning processor 120 includes the six sub-model processors 121 to 126, but the number of sub-model processors included in the deep learning processor 120 may increase according to the number of pieces of image data being used. The sub-model processors 121 to 126 of the present disclosure may be configured as different pieces of hardware but are not limited thereto. The sub-model processors 121 to 126 may be configured as one processor and distinguished by output sub-features.

The first sub-model processor 121 may extract a first sub-feature F21 from first image data D21. The second sub-model processor 122 may extract a second sub-feature F22 from second image data D22. The third sub-model processor 123 may extract a third sub-feature F23 from third image data D23. The fourth sub-model processor 124 may extract a fourth sub-feature F24 from fourth image data D24. The fifth sub-model processor 125 may extract a fifth sub-feature F25 from fifth image data D25. The sixth sub-model processor 126 may extract a sixth sub-feature F26 from sixth image data D26.

The first to sixth image data D21 to D26 may correspond to a captured fundus image, a corneal endothelial cell image, a corneal shape and aberration analyzer (e.g., Keratron Scout) image, an optical coherence tomography image, an OPD-scan III image, and a computed corneal tomography machine (e.g., Pentacam AXL) image, respectively. Each of the first to sixth sub-model processors 121 to 126 may include a convolution-based deep neural network. The first to sixth sub-features F21 to F26 indicate numbers or arrays of numbers.

The sub-fusion processor 127 may extract the second feature F20 from the first to sixth sub-features F21 to F26. The sub-fusion processor 127 may extract the second feature F20 on the basis of, for example, a model including a fully connected layer.

FIG. 4 is a flowchart of a myopic regression prediction method 200 according to embodiments of the present disclosure.

Referring to FIGS. 4 and 1 together, the myopic regression prediction method 200 may include an operation S200 of predicting the at least one R of a probability of myopic regression and whether myopic regression will occur from the numerical data D10 including a refractive power, a corneal curvature, an eye axial length, a photopic pupil size, a mesopic pupil size, a corneal diameter, a corneal thickness, a corneal epithelial thickness, a high-order aberration, a visual acuity, an intraocular pressure, a sex, and an age, using the machine learning processor 110.

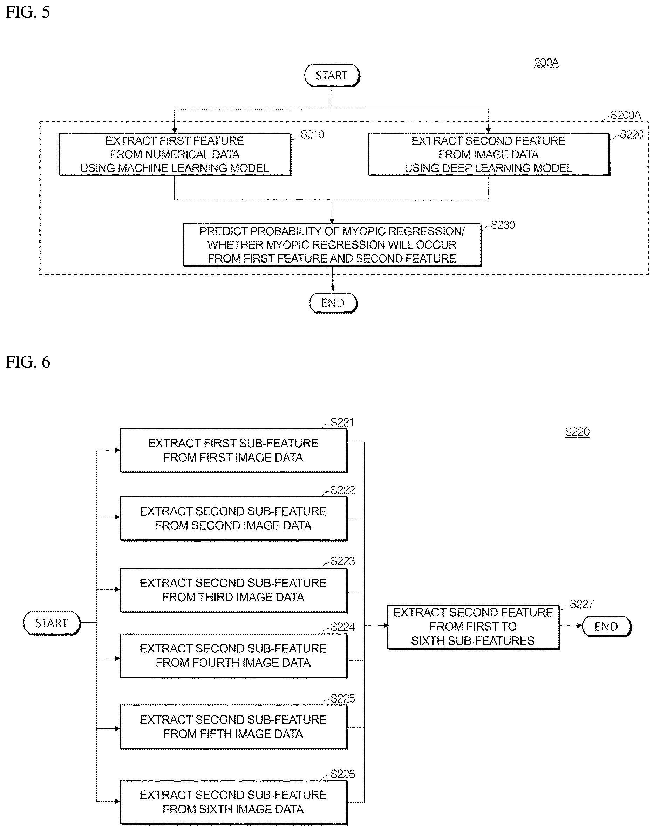

FIG. 5 is a flowchart of a myopic regression prediction method 200A according to embodiments of the present disclosure.

Referring to FIGS. 5 and 2 together, the myopic regression prediction method 200A may include an operation S200A of predicting the at least one R of a probability of myopic regression and whether myopic regression will occur from the numerical data D10 using the machine learning processor 110. The operation S200A of predicting the at least one R of a probability of myopic regression and whether myopic regression will occur from the numerical data D10 using the machine learning processor 110 may further use the deep learning processor 120 and the image data D20 including a captured fundus image, a corneal endothelial cell image, a corneal shape and aberration analyzer image, an optical coherence tomography image, an OPD-scan III image, and a computed corneal tomography machine image.

According to some embodiments, the operation S200A of predicting the at least one R of the probability of myopic regression and whether myopic regression will occur from the numerical data D10 using the machine learning processor 110 may include an operation S210 of extracting the first feature F10 from the numerical data D10 using the machine learning processor 110, an operation S220 of extracting the second feature F20 from the image data D20 using the deep learning processor 120, and an operation of predicting the at least one R of the probability of myopic regression and whether myopic regression will occur from the first feature F10 and the second feature F20 using the fusion processor 130. According to some exemplary embodiments, the operation S210 of extracting the first feature F10 and the operation S220 of extracting the second feature F20 may be simultaneously performed. According to another embodiment, the operation S220 of extracting the second feature F20 may be performed after the operation S210 of extracting the first feature F10. According to another embodiment, the operation S220 of extracting the second feature F20 may be performed before the operation S210 of extracting the first feature F10.

FIG. 6 is a flowchart of the operation S220 of extracting a second feature from image data using a deep learning processor according to embodiments of the present disclosure.

Referring to FIGS. 6 and 3 together, the operation S220 of extracting a second feature may include an operation S221 of extracting a first sub-feature F21 from the first image data D21 using the first sub-model processor 121, an operation S222 of extracting a second sub-feature F22 from the second image data D22 using the second sub-model processor 122, an operation S223 of extracting a third sub-feature F23 from the third image data D23 using the third sub-model processor 123, an operation S224 of extracting a fourth sub-feature F24 from the fourth image data D24 using the fourth sub-model processor 124, an operation S225 of extracting a fifth sub-feature F25 from the fifth image data D25 using the fifth sub-model processor 125, an operation S226 of extracting a sixth sub-feature F26 from the sixth image data D26 using the sixth sub-model processor 126, and an operation S227 of extracting the second feature F20 from the first to sixth sub-features F21 to F26.

Although the six sub-feature extraction operations S221 to S226 are shown in FIG. 6, the number of sub-feature extraction operations may increase according to the number of pieces of image data being used. According to some embodiments, the sub-feature extraction operations S221 to S226 may be simultaneously performed in parallel. According to another embodiment, the sub-feature extraction operations S221 to S226 may be performed in sequence. In other words, the order of performing the sub-feature extraction operations S221 to S226 may be variously changed.

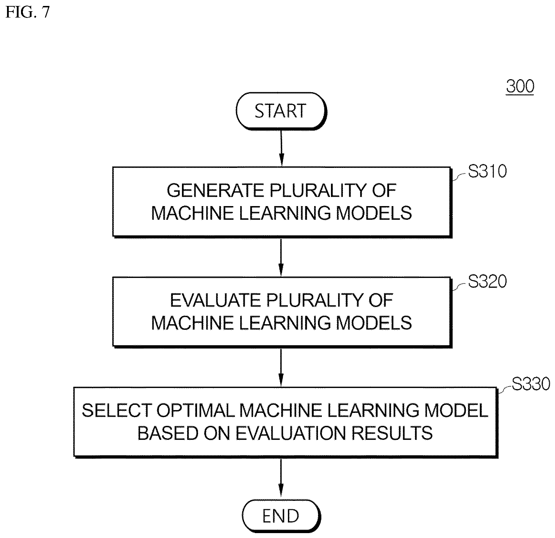

FIG. 7 is a flowchart of a method 300 of training a myopic regression prediction medical apparatus according to embodiments of the present disclosure.

Referring to FIG. 7, the method 300 of training a myopic regression prediction medical apparatus may include an operation S310 of generating a plurality of machine learning processors, an operation S320 of evaluating the plurality of machine learning processors, and an operation S330 of selecting an optimal machine learning processor on the basis of evaluation results.

According to an embodiment, the operation S310 of generating the plurality of machine learning processors may include an operation of processing a missing value, selecting a preprocessing method, selecting a machine learning method, and selecting a postprocessing method. In other words, at least one of a missing value processing method, a preprocessing method, a machine learning method, and a postprocessing method of the plurality of machine learning processors may differ from the others. The method 300 of training a myopic regression prediction medical apparatus may select at least one technique among a missing value processing method, a preprocessing method, a machine learning method, and a postprocessing method each including a plurality of techniques, thereby generating a machine learning processor including various combinations of a missing value processing method, a preprocessing method, a machine learning method, and a postprocessing method.

For example, a missing value processing method may be do nothing, K-NN, MICE, missForest, AutoEncoder, GAIN, EM, or a combination thereof.

For example, a preprocessing method may be standardization, normalization, PCA, fast ICA, or a combination thereof.

For example, a machine learning method may be linear regression, logistic regression, SVM, decision tree, random forest, XGBoost, or deep learning. According to some embodiments, a machine learning method may include an ensemble, that is, a combination, of a plurality of machine learning methods.

For example, a postprocessing method may be no postprocessing, sigmoid, isotonic, or a combination thereof.

In the operation S320 of evaluating the plurality of machine learning processors, for example, mean squared error (MSE), accuracy, precision, recall, F1-score, etc. may be used.

In the operation S330 of selecting the optimal machine learning processor on the basis of the evaluation results, machine learning processing showing the highest performance in evaluation results may be selected.

According to some embodiments, the method 300 of training a myopic regression prediction medical apparatus may be performed using AutoMachineLearning (AutoML), MachineLearningOperations (MLOps), or a combination thereof. When AutoML, MLOps, or a combination thereof is used, it is possible to easily perform the operation S310 of generating a plurality of machine learning processors, the operation S320 of evaluating the plurality of machine learning processors, and the operation S330 of selecting an optimal machine learning processor on the basis of evaluation results, and thus efficiency in developing a myopic regression prediction medical apparatus can be increased.

The various embodiments described above can be implemented as software including instructions stored in a recording medium which can be read by a computer or a similar device using software, hardware, or a combination thereof. The device-readable recording medium may be provided in the form of a non-transitory storage medium. Here, "non-transitory" means that the storage medium does not include a signal and is tangible, and does not distinguish between a case in which data is stored in the storage medium semi-permanently and a case in which data is temporarily stored.

Exemplary embodiments have been disclosed as described above in the drawings and specification. However, it is to be understood that the exemplary embodiments are not intended to limit the technical spirit of the present invention but are intended to better describe the present invention to those of ordinary skill in the art. The true technical scope of the present disclosure should be determined by the technical scope of the appended claims.

## Claims

1. A medical apparatus for predicting myopic regression which is configured to predict at least one of a probability of myopic regression and whether myopic regression will occur from numerical data including a refractive power, a corneal curvature, an eye axial length, a photopic pupil size, a mesopic pupil size, a corneal diameter, a corneal thickness, a corneal epithelial thickness, a high-order aberration, a visual acuity, an intraocular pressure, a sex, and an age, using a machine learning processor.

2. The medical apparatus of claim 1, wherein, to predict at least one of the probability of myopic regression and whether myopic regression will occur, a deep learning processor and image data including a captured fundus image, a corneal endothelial cell image, a corneal shape and aberration analyzer image, an optical coherence tomography image, an optical path difference (OPD)-scan III image, and a computed corneal tomography machine image are additionally used.

3. The medical apparatus of claim 2, wherein the machine learning processor is configured to extract a first feature from the numerical data, and
the deep learning processor is configured to extract a second feature from the image data,
the medical apparatus further comprising a fusion processor configured to predict at least one of the probability of myopic regression and whether myopic regression will occur from the first feature and the second feature.

4. The medical apparatus of claim 3, wherein the deep learning processor comprises:
a first sub-model processor configured to extract a first sub-feature from the captured fundus image;
a second sub-model processor configured to extract a second sub-feature from the corneal endothelial cell image;
a third sub-model processor configured to extract a third sub-feature from the corneal shape and aberration analyzer image;
a fourth sub-model processor configured to extract a fourth sub-feature from the optical coherence tomography image;
a fifth sub-model processor configured to extract a fifth sub-feature from the OPD-scan III image;
a sixth sub-model processor configured to extract a sixth sub-feature from the computed corneal tomography machine image; and
a sub-fusion processor configured to extract the second feature from the first to sixth sub-features.

5. The medical apparatus of claim 1, wherein the machine learning processor comprises:
a missing value processing processor configured to process a missing value of the numerical data;
a preprocessing processor configured to preprocess the numerical data;
a learning processor configured to extract a feature from the preprocessed numerical data; and
a postprocessing processor configured to predict at least one of the probability of myopic regression and whether myopic regression will occur from the extraction unit.

6. A method of predicting myopic regression, comprising predicting at least one of a probability of myopic regression and whether myopic regression will occur from numerical data including a refractive power, a corneal curvature, an eye axial length, a photopic pupil size, a mesopic pupil size, a corneal diameter, a corneal thickness, a corneal epithelial thickness, a high-order aberration, a visual acuity, an intraocular pressure, a sex, and an age, using a machine learning processor.

7. The method of claim 6, wherein the predicting of at least one of the probability of myopic regression and whether myopic regression will occur further uses a deep learning processor and image data including a captured fundus image, a corneal endothelial cell image, a corneal shape and aberration analyzer image, an optical coherence tomography image, an optical path difference (OPD)-scan III image, and a computed corneal tomography machine image.

8. The method of claim 7, wherein the predicting of at least one of the probability of myopic regression and whether myopic regression will occur comprises:
extracting a first feature from the numerical data using the machine learning processor;
extracting a second feature from the image data using the deep learning processor; and
predicting at least one of the probability of myopic regression and whether myopic regression will occur from the first feature and the second feature.

9. The method of claim 8, wherein the extracting of the second feature comprises:
extracting a first sub-feature from the captured fundus image;
extracting a second sub-feature from the corneal endothelial cell image;
extracting a third sub-feature from the corneal shape and aberration analyzer image;
extracting a fourth sub-feature from the optical coherence tomography image;
extracting a fifth sub-feature from the OPD-scan III image;
extracting a sixth sub-feature from the computed corneal tomography machine image; and
extracting the second feature from the first to sixth sub-features.

10. A method of training a myopic regression prediction medical apparatus configured to predict at least one of a probability of myopic regression and whether myopic regression will occur from numerical data including a refractive power, a corneal curvature, an eye axial length, a photopic pupil size, a mesopic pupil size, a corneal diameter, a corneal thickness, a corneal epithelial thickness, a high-order aberration, a visual acuity, an intraocular pressure, a sex, and an age, the method comprising:
generating, by at least one of a missing value processing method, a preprocessing method, and a postprocessing method, a plurality of different machine learning processors;
training the plurality of machine learning processors;
evaluating the plurality of machine learning processors; and
selecting an optimal machine learning processor among the plurality of machine learning processors on the basis of evaluation results.
